# EUROPÄISCHE PATENTSCHRIFT

(11) **EP 3 829 488 B1**
(45) Veröffentlichungstag und Bekanntmachung des Hinweises auf die Patenterteilung: **06.04.2022**
(21) Anmeldenummer: 19742342.9
(22) Anmeldetag: 17.07.2019
(51) Int. Cl.: A61F 2/16

(54) **AKKOMODATIVE INTRAOKULARLINSE**
ACCOMMODATIVE INTRAOCULAR LENS
LENTILLE INTRAOCULAIRE ACCOMMODATIVE

(30) Priorität: 31.07.2018 DE 102018212774
(43) Veröffentlichungstag der Anmeldung: 09.06.2021
(73) Patentinhaber: Carl Zeiss Meditec AG, 07745 Jena (DE)
(72) Erfinder: LUQUE, Sergio Oscar, 1220 Wien (AT); RICHTER, Frank, 14482 Potsdam (DE); BUCHHEISTER, Jan, 07751 Jena (DE); WOLF, Uwe, 99441 Magdala (DE)
(74) Vertreter: Carl Zeiss AG - Patentabteilung
(86) Internationale Anmeldenummer: PCT/EP2019/069254
(87) Internationale Veröffentlichungsnummer: WO 2020/025325

(56) Entgegenhaltungen:
- US-A1- 2008 004 699
- US-A1- 2014 180 403
- US-A1- 2016 157 996
- US-A1- 2017 172 732

## Beschreibung

Die Erfindung betrifft eine akkommodative Intraokularlinse.

Mit einer natürlichen Augenlinse ist es möglich, in der Ferne und in der Nähe Gegenstände scharf zu sehen. Ermöglicht wird dies dadurch, dass die Augenlinse in ihrer Form und damit die Brechkraft verändert werden kann. Die Augenlinse ist in einem Kapselsack enthalten, der an Zonulafasern aufgehängt ist, welche wiederum mit einem Ziliarmuskel verbunden sind. Wenn der Ziliarmuskel sich entspannt, werden die Zonulafasern gestrafft, wodurch der Kapselsack gestreckt wird. Die Formänderung des Kapselsackes bewirkt bei einer weichen Augenlinse, dass diese sich ebenfalls in ihrer Form verändert. Mit zunehmender Streckung des Kapselsackes wird die Augenlinse zunehmend abgeflacht. Dadurch ändert sich die Brechkraft der Augenlinse. Eine abgeflachte Augenlinse führt zu einer geringeren Brechkraft, sodass eine scharfe Fernsicht ermöglicht wird. Dieser Vorgang ist reversibel, sodass bei einem angespannten Ziliarmuskel die Zonulafasern erschlaffen und der Kapselsack weniger stark gestreckt ist. Damit nimmt die Augenlinse eine Form an, die stärker gewölbt ist, sodass eine höhere Refraktion erreicht wird. Dadurch ist es möglich, Gegenstände in der Nähe scharf zu sehen. Diese Variation in der Schärfenebene wird als Akkommodation bezeichnet.

Es ist normal, dass mit zunehmendem Alter die Augenlinse an Elastizität verliert. Die Augenlinse ist dann weniger in der Lage, ihre Form als Reaktion auf eine Kontraktion des Ziliarmuskels zu ändern. Dies bewirkt, dass ein Fokussieren auf nahe Gegenstände immer schwerer gelingt. Dieser Zustand wird mit Presbyopie bezeichnet. Durch das Tragen einer Brille oder einer Kontaktlinse ist es möglich, die fehlende Brechkraft zu kompensieren. Mit zunehmendem Alter wird die Augenlinse jedoch zunehmend inelastischer bis hart und kann zudem eintrüben. In der Medizin wird eine solcher Zustand der Augenlinse mit Katarakt oder "grauer Star" bezeichnet. Ein Brillenglas kann die Folgen einer Eintrübung der Augenlinse nicht kompensieren, sodass es üblich geworden ist, die eingetrübte Augenlinse chirurgisch zu entfernen. Dazu wird eine mit Ultraschall schwingende Nadel in das Auge eingeführt und die harte und eingetrübte Augenlinse in kleine Partikel zertrümmert. Dieser Vorgang wird als Phakoemulsifikation bezeichnet. Die Partikel werden nach einer solchen Phakoemulsifikation abgesaugt, bis der Kapselsack von der natürlichen Augenlinse befreit ist. Um wieder ein gutes Sehen zu ermöglichen, wird anschließend eine künstliche Augenlinse in den Kapselsack implantiert. Diese künstliche Augenlinse wird als Intraokularlinse bezeichnet.

Die künstliche Augenlinse ist üblicherweise eine Linse mit einem einzigen Brennpunkt (monofokal), sodass ein Patient nach einer Implantation einer künstlichen Augenlinse eine Brille oder eine Kontaktlinse benötigt, um sowohl in der Ferne als auch in der Nähe scharf zu sehen. Es gibt jedoch auch Überlegungen, die künstliche Augenlinse so zu gestalten, dass eine Akkommodation mit einer sich verändernden Schärfenebene möglich ist. Das Anspannen oder Entspannen eines Ziliarmuskels soll es ermöglichen, die Brechkraft der Intraokularlinse zu verändern. In US 2012/0 296 424 A1 ist eine solche akkommodative Intraokularlinse beschrieben. Nachteilig daran ist, dass eine solche Intraokularlinse sehr komplex aufgebaut ist und eine aufwändige Implantation erfordert. Hinzu kommt, dass ein natürlicher Kapselsack bei jedem Menschen eine andere Größe aufweist, sodass eine derartige akkommodative Intraokularlinse bei einigen Menschen für den vorhandenen Kapselsack zu groß oder zu klein ist und somit entweder zu eng oder zu schlaff im Kapselsack angeordnet ist. Eine akkommodative Intraokularlinse mit einem einfacheren Aufbau ist zum Beispiel in US 6 197 059 B1 beschrieben. Die Intraokularlinse weist einen Optikkörper und eine daran gekoppelte Haptik auf, wobei die Haptik so biegsam ist, dass als Reaktion auf die Bewegung des Ziliarmuskels der Optikkörper entlang der optischen Achse des Optikkörpers vor oder zurück bewegt werden kann. Nachteilig daran ist jedoch, dass die erreichbare Akkommodation relativ gering ist.

Weitere akkommodative Intraokularlinsen sind in US 2008 / 0 004 699 A1, US 2017/0172732 A1 und in US 6 443 985 B1 beschrieben.

Es ist eine Aufgabe der Erfindung, eine akkommodative Intraokularlinse zu schaffen, welche einen einfachen Aufbau aufweist, mittels Mikroinzision implantiert werden kann, einen großen Akkommodationsbereich ermöglicht und bei einem Patienten mit einem kleinen oder einem großen Kapselsack gleichermaßen gut darin angeordnet werden kann.

Die Aufgabe wird durch den Gegenstand des Anspruchs 1 gelöst. Vorteilhafte Weiterbildungen der Erfindung sind Gegenstand der Unteransprüche.

Die akkommodative Intraokularlinse zur Implantation in ein Auge innerhalb eines natürlichen Kapselsackes im Auge, der an seinem Umfang mittels Zonulafasern an einem Ziliarmuskel des Auges angebracht ist, weist auf:
- einen ersten Linsenteil, welcher aufweist:
   - einen für Licht transparenten Optikkörper mit einer optischen Achse, einer vorderen Optikkörperoberfläche und einer hinteren Optikkörperoberfläche,
   - eine mit dem Optikkörper fest verbundene Haptik, welche eingerichtet ist, mit dem Kapselsack in Eingriff zu kommen, um den Optikkörper im Kapselsack mittig anzuordnen,
   - eine mit der Haptik oder dem Optikkörper fest verbundene flexible Membran, welche eine vordere Membranoberfläche und eine hintere Membranoberfläche aufweist, wobei die Membran benachbart zur vorderen Optikkörperoberfläche angeordnet ist, wobei die Membran eine Mittelachse aufweist, die kongruent oder parallel zur optischen Achse verläuft, wobei die hintere Membranoberfläche einen Krümmungsradius aufweist, wobei die Membran für Licht transparent ist, und
- einen zweiten Linsenteil, welcher einen Hohlzylinder aufweist,
wobei sich der Hohlzylinder mit einem proximalen Ende auf die vordere Membranoberfläche des ersten Linsenteils so platzieren lässt, dass durch eine auf ein distales Ende des Hohlzylinders parallel zur optischen Achse wirkende Druckkraft, welche durch eine Bewegung des Ziliarmuskels des Auges erzeugbar ist, der Hohlzylinder und die Membran entlang der optischen Achse in Richtung zur vorderen Optikkörperoberfläche hin verlagerbar sind und dabei die hintere Membranoberfläche eine Änderung Ihres Krümmungsradius erfährt, wobei sich der Hohlzylinder mit der Membran lösbar koppeln lässt.

Die erfindungsgemäße Intraokularlinse weist somit einen ersten Linsenteil und einen zweiten Linsenteil auf, wobei sich der zweite Linsenteil lösbar mit dem ersten Linsenteil koppeln lässt. Der erste Linsenteil weist einen Optikkörper und eine Haptik auf, was bei jeder monofokalen Intraokularlinse üblich ist. Die zusätzliche Membran ist eine Ergänzung, die durch thermische oder chemische Einwirkung mit dem Optikkörper oder der Haptik fest verbunden werden kann, sodass der Optikkörper, die Haptik und die Membran einstückig ausgebildet sind. Die Membran ist relativ einfach herstellbar und nimmt wenig Platz in Anspruch. Ein solcher erster Linsenteil kann daher wie üblich mittels einer Mikroinzision in ein Auge implantiert werden. Mit einer Mikroinzision ist hier gemeint, dass in eine Hornhaut eines Auges und den dahinter angeordneten Kapselsack eine Spitze eines Injektors durch eine Öffnung mit einem Durchmesser von maximal 3,0 mm, bevorzugt kleiner als 2, 5 mm und besonders bevorzugt kleiner als 1,8 mm eingeschoben wird, durch welche der erste Linsenteil in den Kapselsack injiziert werden kann. Eine Mikroinzision ist vorteilhaft, da dies nur eine kleine Verletzung des Auges darstellt, sodass eine rasche Erholung des Sehvermögens nach einer Implantation erreichbar ist.

Die hintere Membranoberfläche weist einen Krümmungsradius auf. Im Extremfall kann dieser Krümmungsradius unendlich sein. Die hintere Membranoberfläche besitzt dann die Form einer Ebene. Der Krümmungsradius kann aber auch kleiner als unendlich sein, sodass die hintere Membranoberfläche eine konvexe oder konkave Form annimmt.

Der zweite Linsenteil weist einen Hohlzylinder auf, der sich mit der Membran lösbar koppeln lässt. Dies hat den Vorteil, dass der zweite Linsenteil nicht zusammen mit dem ersten Linsenteil in den Kapselsack injiziert werden muss. Stattdessen ist es möglich, den zweiten Linsenteil zeitlich nach der Implantation des ersten Linsenteils in den Kapselsack zu injizieren. Der zweite Linsenteil besitzt als Hohlzylinder eine noch einfachere Geometrie als der erste Linsenteil und lässt sich einfach falten oder einrollen, sodass eine Mikroinzision des zweiten Linsenteils problemlos möglich ist. Für einen Chirurgen ist es relativ einfach, den zweiten Linsenteil auf die vordere Membranoberfläche des ersten Linsenteils zu platzieren. Ein solcher Aufbau einer akkommodativen Intraokularlinse ist vorteilhaft, da die korrekte Höhe des zweiten Linsenteils durch Vermessen des Kapselsackes vor einer Operation für den zu behandelnden Patienten genau ermittelt werden kann.

Der erste Linsenteil mit seinem Optikkörper wird nach einer Vermessung des Auges des Patienten genau auf diesen Patienten abgestimmt, so dass der Patient ein gutes Sehvermögen, zum Beispiel für die Ferne, erreichen kann. Der zweite Linsenteil ist nach einer Vermessung des Kapselsackes ebenfalls speziell für den zu behandelnden Patienten ausgewählt. Der zweite Linsenteil besitzt somit keine konstante Höhe, die bei jedem Patienten den gleichen Wert besitzt. Die Intraokularlinse ist somit nicht nur im Hinblick auf den Optikkörper und dessen optische Leistung, sondern auch im Hinblick auf den zweiten Linsenteil speziell auf den Patienten abgestimmt.

Nach einem Platzieren des proximalen Endes des Hohlzylinders auf die vordere Membranoberfläche und einer anschließende Bewegung des Ziliarmuskels wird der Kapselsack gestreckt oder entspannt, wodurch sich die Form des Kapselsackes ändert. Da das distale Ende des Hohlzylinders mit einer Innenwand des Kapselsackes in Eingriff steht, wird durch die Änderung der Form des Kapselsackes eine unterschiedlich starke Druckkraft auf den Hohlzylinder und die vordere Membranoberfläche ausgeübt, sodass der Hohlzylinder und die Membran entlang der optischen Achse in Richtung zur vorderen Optikkörperoberfläche hin oder von dieser fort verlagerbar sind. Die unterschiedliche Lage der Membran in Relation zum Optikkörper bewirkt eine Änderung des Krümmungsradius der hinteren Membranoberfläche und damit eine unterschiedliche Refraktion, sodass eine Akkommodation des Auges auf Gegenstände in der Ferne oder in der Nähe erreichbar ist. Die Änderung des Krümmungsradius der hinteren Membranoberfläche mit zunehmender Verlagerung der Membran in Richtung zum Optikkörper bedeutet eine Veränderung des Brennpunktes in Richtung zu einer Fernsicht.

Es wird darauf hingewiesen, dass die Membran an sich keine Refraktion bewirken muss und die vordere Membranoberfläche und die hintere Membranoberfläche bevorzugt planparallel zueinander ausgebildet sind. Die Membran kann damit sehr einfach gefertigt werden. Eine Akkommodation wird bereits dadurch erreicht, dass die Lage der Membran relativ zum Optikkörper veränderbar ist und dabei die hintere Membranoberfläche eine Änderung Ihres Krümmungsradius erfährt. Je näher die Membran an den Optikkörper verlagert wird, umso stärker ist die Änderung des Krümmungsradius der hinteren Membranoberfläche.

Der erste Linsenteil und der zweite Linsenteil sind bevorzugt aus einem Acrylpolymer gebildet. Bevorzugt sind der erste Linsenteil und der zweite Linsenteil aus dem gleichen Acrylpolymer gebildet.

Gemäß einer Ausführungsform der Erfindung ist die Membran hermetisch dicht mit der Haptik oder dem Optikkörper gekoppelt, sodass ein Innenraum zwischen der hinteren Membranoberfläche und der vorderen Optikkörperoberfläche gebildet ist, wobei der Innenraum mit Gas gefüllt ist. Dies ist vorteilhaft, da Gas einen anderen Refraktionsindex als ein aus einem Acrylpolymerwerkstoff gebildeter Optikkörper oder eine Membran aufweist. Der Refraktionsindex eines Acrylpolymers liegt bei etwa 1,47 bis 1,55, der Refraktionsindex eines Gases wie zum Beispiel Luft liegt bei etwa 1,00003, wobei diese Werte bei der Wellenlänge von 589 nm der Natrium-D-Linie gelten. Somit lässt sich durch Einsatz eines Gases im Innenraum eine Differenz des Refraktionsindex von etwa 0,5 erreichen. Wenn sich durch eine Verlagerung des Hohlzylinders und der Membran relativ zum Optikkörper die Höhe des Innenraumes verändert, sodass sich das vorhandene Gasvolumen zwischen der hinteren Membranoberfläche und der vorderen Optikkörperoberfläche verändert, hat dies eine Veränderung der Brechkraft der gesamten Intraokularlinse zur Folge. Bereits durch eine geringe Akkommodation und somit Verlagerung von Hohlzylinder und Membran relativ zum Optikkörper und damit Änderung des Krümmungsradius der hinteren Membranoberfläche kann eine relativ große Veränderung der Refraktion erreicht werden.

Gemäß einer Weiterbildung der Erfindung ist das mit Gas gefüllte Volumen in dem Innenraum auf ein Volumen im Bereich von 3 bis 10 mm³, bevorzugt 4 bis 6 mm³, begrenzt. Dies hat den Vorteil, dass auch bei einem Atmosphärendruck in der Umgebung des Patienten, der von dem für den Patienten sonst üblichen Atmosphärendruck abweicht, zum Beispiel bei einem Aufenthalt im Gebirge oder in einem Flugzeug, eine Ausdehnung des Gases nur zu einer geringen Verlagerung der Membran in Relation zum Optikkörper und damit Änderung des Krümmungsradius der hinteren Membranoberfläche führt.

Bevorzugt weist die Membran Führungselemente auf, mit welchen der Hohlzylinder in Eingriff kommen kann und damit zentrisch zur optischen Achse auf der Membran platzierbar ist. Dies ist vorteilhaft, da sich somit der Hohlzylinder optimal in Relation zur Membran anordnen lässt, wodurch eine optimale Funktion der Intraokularlinse sichergestellt werden kann. Durch die Führungselemente kann eine dauerhaft stabile Lage des Hohlzylinders in Relation zur Membran erreicht werden.

Gemäß einer Ausführungsform der Erfindung weist die Membran einen zentrischen Bereich und einen peripheren Bereich auf, wobei der zentrische Bereich eine größere Dicke als der periphere Bereich aufweist. Damit wird erreicht, dass der periphere Bereich die Funktion eines Filmgelenkes einnimmt und eine Änderung des Krümmungsradius der hinteren Membranoberfläche zuverlässig und mit geringer Kraft möglich ist.

Zudem ist es möglich, dass die Membran so verlagerbar ist, dass sich die hintere Membranoberfläche mindestens mit einem Scheitelpunkt der vorderen Optikkörperoberfläche in Kontakt bringen lässt. Damit gibt es einen ersten Zustand, in dem die Membran nicht mit der vorderen Optikkörperoberfläche in Kontakt ist, und einen zweiten Zustand, in dem die Membran mit der vorderen Optikkörperoberfläche in Kontakt ist. Dies ist vorteilhaft, da sich auf diese Weise ein deutlicher Unterschied in der Refraktion der gesamten Intraokularlinse erreichen lässt. Wenn durch zunehmende Druckkraft auf das distale Ende des Hohlzylinders die Membran nicht nur mit dem Scheitelpunkt, sondern mit einem geschlossenen Bereich der vorderen Optikkörperoberfläche in Kontakt kommt, kommt es zu einer deutlichen Änderung der Refraktion.

Bevorzugt weist der Hohlzylinder an dem distalen Ende einen umlaufenden Kragen auf, der sich mit einer Innenwand des Kapselsackes in Eingriff bringen lässt. Damit wird ein größerer Flächenkontakt mit der Innenwand des Kapselsackes erreicht. Dies ist vorteilhaft, da somit eine sehr stabile Lage des Hohlzylinders im Kapselsack möglich ist.

Gemäß einer Weiterbildung der Erfindung ist auf der vorderen Optikkörperoberfläche eine Erhebung ausgebildet, welche eine Höhe aufweist, die größer als 0,05 mm ist. Die Erhebung kann die Form einer Halbkugel, eines Ringes oder eines Ringsegmentes aufweisen. Wenn diese Erhebung eine Höhe von größer als 0,05 mm aufweist, kann bei einer Verlagerung der Membran in Richtung zur vorderen Optikkörperoberfläche und damit einhergehenden Änderung des Krümmungsradius der hinteren Membranoberfläche eine Flächenberührung der hinteren Membranoberfläche mit der vorderen Optikkörperoberfläche verhindert werden. Wenn die hintere Membranoberfläche und die vordere Optikkörperoberfläche jeweils nur eine geringe Rauheit besitzen, lässt sich mittels der Erhebung ein großflächiges Anhaften, z.B. durch Adhäsion der beiden Oberflächen miteinander, verhindern.

Weitere Vorteile und Merkmale der Erfindung werden mit Bezug auf die nachfolgenden Zeichnungen erklärt, in welchen zeigen:
- Fig. 1: eine schematische perspektivische Querschnittsdarstellung einer Ausführungsform eines ersten Linsenteils der erfindungsgemäßen Intraokularlinse;
- Fig. 2: eine schematische perspektivische Querschnittsdarstellung einer Ausführungsform eines zweiten Linsenteils der erfindungsgemäßen Intraokularlinse;
- Fig. 3: eine schematische Querschnittsdarstellung einer ersten Ausführungsform der erfindungsgemäßen Intraokularlinse in einem Kapselsack eines Auges, wenn die Zonulafasern nicht gespannt sind;
- Fig. 4: eine schematische Querschnittsdarstellung der erfindungsgemäßen Intraokularlinse gemäß Fig. 3 in einem Kapselsack eines Auges, wenn die Zonulafasern gespannt sind;
- Fig. 5: eine schematische perspektivische Querschnittsdarstellung einer weiteren Ausführungsform des ersten Linsenteils der erfindungsgemäßen Intraokularlinse mit Erhebungen auf einer Oberfläche eines Optikkörpers;
- Fig. 6: eine schematische Querschnittsdarstellung der erfindungsgemäßen Intraokularlinse gemäß Fig. 5 in einem Kapselsack eines Auges, wenn die Zonulafasern gespannt sind,
- Fig. 7: eine schematische Querschnittsdarstellung einer weiteren Ausführungsform der erfindungsgemäßen Intraokularlinse in einem Kapselsack eines Auges, wenn die Zonulafasern nicht gespannt sind; und
- Fig. 8: eine schematische Querschnittsdarstellung der erfindungsgemäßen Intraokularlinse gemäß Fig. 7 in einem Kapselsack eines Auges, wenn die Zonulafasern gespannt sind.

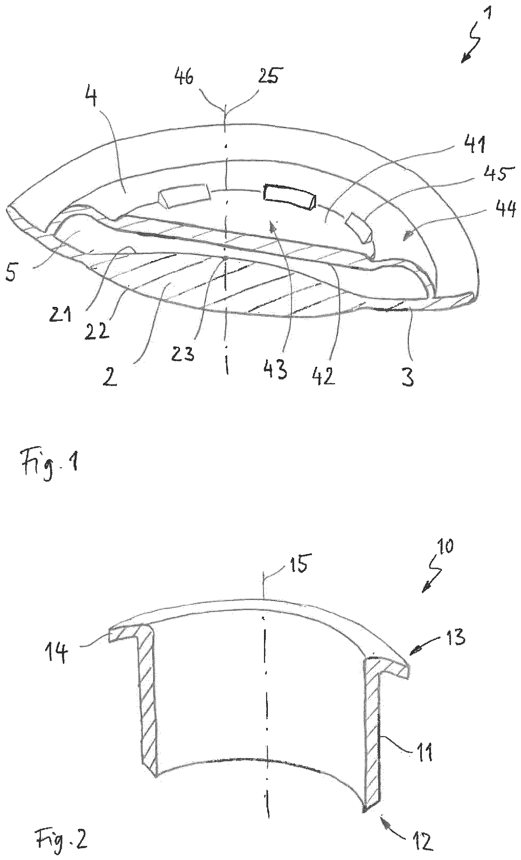

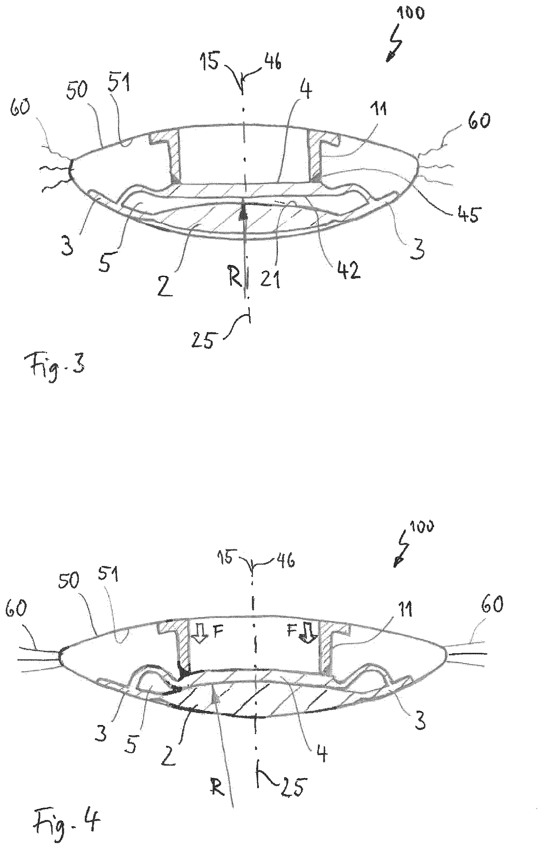

In Fig. 1 ist eine schematische perspektivische Querschnittsdarstellung einer Ausführungsform eines ersten Linsenteils 1 der erfindungsgemäßen Intraokularlinse 100 dargestellt. Der erste Linsenteil 1 weist einen für Licht transparenten Optikkörper 2 mit einer vorderen Optikkörperoberfläche 21 und einer hinteren Optikkörperoberfläche 22 auf. Der Optikkörper 2 ist bei dieser Ausführungsform rotationssymmetrisch um eine optische Achse 25 ausgebildet. Mit dem Optikkörper 2 ist eine Haptik 3 fest verbunden und einstückig ausgebildet. Die Haptik 3 ist dazu eingerichtet, mit einem Kapselsack 50 in Eingriff zu kommen, um den Optikkörper 2 im Kapselsack 50 mittig anzuordnen, siehe Fig. 3.

Mit der Haptik 3 oder dem Optikkörper 2 ist eine flexible Membran 4 fest verbunden, wobei bei der in Fig. 1 gezeigten Ausführungsform die Membran 4 mit der Haptik 3 einstückig ausgebildet ist. Die Membran 4 weist eine vordere Membranoberfläche 41 und eine hintere Membranoberfläche 42 auf und ist benachbart zur vorderen Optikkörperoberfläche 21 angeordnet. Die Membran 4 weist eine Mittelachse 46 auf, die bei dieser Ausführungsform kongruent zur optischen Achse 25 des Optikkörpers 2 verläuft, wobei die Membran 4 aus einem Werkstoff gebildet ist, der für Licht transparent ist. Die Membran 4 weist einen zentrischen Bereich 43 und einen peripheren Bereich 44 auf, wobei der zentrische Bereich 43 eine größere Dicke als der periphere Bereich 44 aufweist. Im zentrischen Bereich 43 sind die vordere Membranoberfläche 41 und die hintere Membranoberfläche 42 planparallel zueinander ausgebildet. Der periphere Bereich 44 der Membran 4 ist ringförmig mit der Haptik verbunden und gewölbt ausgeführt, wodurch er ein oberes Segment eines Torus bildet. Dadurch wird eine Verlagerung des zentrischen Bereiches 43 entlang der Mittelachse 46 der Membran 4 ermöglicht. Bei einer solchen Ausführungsform weist der periphere Bereich 44 der Membran 4 eine federnde Eigenschaft bei einer Bewegung der Membran 4 entlang der Mittelachse 46 auf. Die vordere Membranoberfläche 41 ist mit Führungselementen 45 versehen, welche mit dem zweiten Linsenteil 10 in Eingriff kommen können.

Das zweite Linsenteil 10, siehe Fig. 2, weist einen Hohlzylinder 11 auf, der rotationssymmetrisch um eine Mittelachse 15 ausgebildet ist. Der Hohlzylinder 11 ist mit einem proximalen Ende 12 versehen, welches mit den Führungselementen 45 der Membran 4 des ersten Linsenteils 1 formschlüssig in Eingriff kommen kann. Am gegenüberliegenden distalen Ende 13 des Hohlzylinders 11 ist ein umlaufender Kragen 14 vorgesehen.

Wenn das erste Linsenteil 1 in einen Kapselsack 50 eines Auges implantiert ist, siehe Fig. 3, kann nachfolgend das zweite Linsenteil 10 in den Kapselsack implantiert werden. Die Haptik 3 ist so ausgebildet, dass sie eine Innenwand 51 des Kapselsackes 50 berührt und damit den Optikkörper 2 im Kapselsack 50 so ausrichtet, dass die optische Achse 25 des Optikkörpers 2 mit der Mittelachse des Kapselsackes 50 im Wesentlichen kongruent verläuft. Das zweite Linsenteil 10 kann dann so auf die vordere Membranoberfläche 41 platziert werden, dass die Mittelachse 15 des Hohlzylinders 10 mit der optischen Achse 25 des Optikkörpers kongruent verläuft. Der Kragen 14 des Hohlzylinders 11 liegt an der Innenwand 51 des Kapselsackes 5 an und überträgt eine Druckkraft F, vgl. Fig. 4, auf die Membran 4, wenn Zonulafasern 60 des Auges gestrafft werden und dadurch der Kapselsack 50 in seiner Querschnittsform abgeflacht wird.

Fig. 3 zeigt den Kapselsack 50 in einem Zustand, in dem die Zonulafasern schlaff sind und somit die Querschnittsform des Kapselsackes 50 relativ ballig ausgebildet ist. In diesem Zustand kann eine Fokussierung im Nahbereich erreicht werden. Fig. 4 zeigt den Kapselsack in einem Zustand, in dem die Zonulafasern 60 gestrafft sind, sodass auf den Kragen 14 am distalen Ende 13 des Hohlzylinders 11 eine Druckkraft F parallel zur optischen Achse 25 wirkt, welche den Hohlzylinder 11 und die Membran 4 in Richtung zur vorderen Optikkörperoberfläche 21 verlagern. Dadurch kann eine Fokussierung im Fernbereich erreicht werden.

Bei der in Fig. 3 dargestellten Ausführungsform befindet sich im Innenraum 5 relativ viel Gas zwischen der hinteren Membranoberfläche 42 und der vorderen Optikkörperoberfläche 21. Bei einer Akkommodation und einem entsprechend abgeflachten Kapselsack 50 gemäß Fig. 4 ist das Gas unter den peripheren Bereich 44 der Membran 4 verlagert, sodass zwischen der hinteren Membranoberfläche 42 und der vorderen Optikkörperoberfläche 21 nur noch sehr wenig oder gar kein Gas vorhanden ist. Dies bewirkt eine deutliche Veränderung der Refraktion, welche durch die Verformung der Membran 4 und teilweise flächige Anlage der hinteren Membranoberfläche 42 an die vordere Optikkörperoberfläche 21 unterstützt wird.

In Fig. 3 ist ferner ein Krümmungsradius R eingezeichnet, der den Krümmungsradius der hinteren Membranoberfläche 42 anzeigt. Bei der in Fig. 3 dargestellten Ausführungsform ist der Krümmungsradius R unendlich, da die hintere Membranoberfläche 42 eine ebene Fläche bildet. Bei dem in Fig. 4 dargestellten Zustand, in dem die Membran relativ stark gewölbt ist und zum Teil flächig an der vorderen Optikkörperoberfläche 21 anliegt, ist der Krümmungsradius kleiner als unendlich. Bei einer flächigen Anlage der hinteren Membranoberfläche 42 mit der vorderen Optikkörperoberfläche 21 ist somit der Krümmungsradius der hinteren Membranoberfläche 42 identisch mit einem Krümmungsradius der vorderen Optikkörperoberfläche 21.

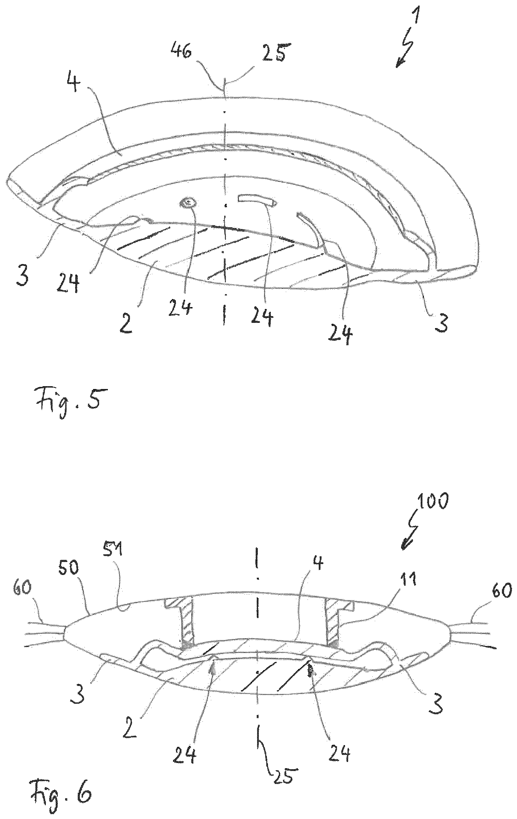

In Fig. 5 ist eine schematische perspektivische Querschnittsdarstellung einer weiteren Ausführungsform des ersten Linsenteils der erfindungsgemäßen Intraokularlinse dargestellt. Die vordere Optikkörperoberfläche 21 ist mit Erhebungen 24 versehen. Eine Erhebung 24 kann zum Beispiel die Form einer Halbkugel oder eines Ringsegmentes oder eines vollständig geschlossenen Ringes aufweisen. Damit ist es möglich, dass bei einer relativ großen Verlagerung der Membran 4 in Richtung zum Optikkörper 2 eine großflächige Berührung und somit ein eventuelles Anhaften zwischen der hinteren Membranoberfläche 42 und der vorderen Optikkörperoberfläche 21 vermieden wird. Die Erhebungen 24 bilden somit ein Lager, welches nur einen Punkt- oder Linienkontakt zwischen der hinteren Membranoberfläche 42 und der vorderen Optikkörperoberfläche 21 erlaubt, vgl. Fig. 6.

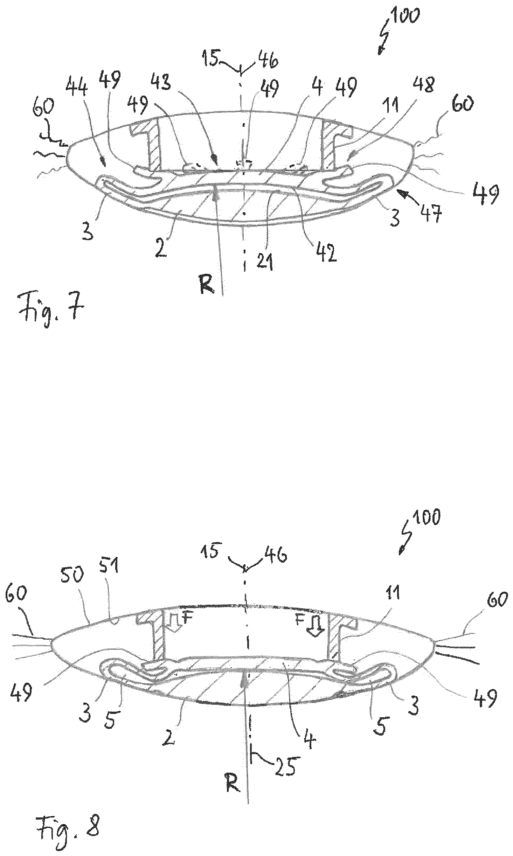

In Fig. 7 ist eine weitere Ausführungsform der Erfindung in einer Querschnittsansicht dargestellt. Die Membran 4 weist einen zentrischen Bereich 43 und einen peripheren Bereich 44 auf. Die Haptik 3 und der periphere Bereich 44 der Membran 4 sind nicht nur einstückig ausgebildet, sondern sind im Querschnitt entlang einer geschwungenen Linie 47 miteinander verbunden, welche im Querschnitt U-förmig ausgebildet ist. Es ist jedoch auch möglich, dass der periphere Bereich 44 der Membran 4 ohne eine im Querschnitt U-förmige Ausbildung direkt mit der Haptik 3 oder dem Optikkörper 2, insbesondere der vorderen Optikkörperoberfläche 21, verbunden ist. Entlang eines Außenrandes 48 des zentrischen Bereiches 43 der Membran 4 sind radial nach außen gerichtet mehrere Biegeelemente 49 in Form eines einseitig fixierten Biegebalkens vorgesehen. Bevorzugt sind die Biegeelemente 49 in gleichem Horizontalwinkel oder Azimut zueinander angeordnet. Der Hohlzylinder 11 ist derart ausgebildet, dass sein proximales Ende 12 auf den Biegeelementen 49 aufsitzen kann.

Wenn die Zonulafasern 60 schlaff sind und die Querschnittsform des Kapselsackes 50 relativ ballig ausgebildet ist, vgl. Fig. 7, weist die hintere Membranoberfläche 42 einen maximalen Radius R auf, welcher kleiner als unendlich ist. Im schlaffen Zustand der Zonulafasern 60 sitzt der Hohlzylinder 11 mit seinem proximalen Ende 12 nur auf den Biegeelementen 49 auf, ohne eine signifikante Durchbiegung der Biegeelemente 49 oder der Membran 4 zu bewirken. Sind die Zonulafasern 60 gestrafft, vgl. Fig. 8, sodass auf den Kragen 14 am distalen Ende 13 des Hohlzylinders 11 eine Druckkraft F parallel zur optischen Achse 25 in Richtung zum Optikkörper 2 wirkt, verformt der Hohlzylinder 11 mittels seines proximalen Endes 12 die Biegeelemente 49 in Richtung zum peripheren Bereich 44 der Membran 4. Dies bewirkt, dass der zentrische Bereich 43 der Membran 4 eine geringere Wölbung erfährt und abgeflacht wird. Der Radius R der hinteren Membranoberfläche 42 vergrößert sich dadurch, wobei dies bevorzugt in einer Membranzone erfolgt, welche nahe der Mittelachse 46 der Membran 4 ist. In einer solchen Membranzone kann der maximale Radius R der hinteren Membranoberfläche 42 unendlich sein. Dies hat zur Folge, dass die Brechkraft der Intraokularlinse 100 abnimmt und eine Fokussierung im Fernbereich erreicht wird. Diese Wirkung kann verstärkt werden, indem in einem Bereich zwischen der hinteren Membranoberfläche 42 und der vorderen Optikkörperoberfläche 21 ein Fluid wie zum Beispiel ein Öl, bevorzugt Silikonöl, ein Gel, bevorzugt ein Silikongel mit einer Shore-Härte im Bereich von 1 (Durometer Typ OOO) bis 100 (Durometer Typ OO), oder ein Gas enthalten ist. Durch die Bewegung des Hohlzylinders 11 in Richtung zum Optikkörper 2 kann dieses Fluid in Richtung zum Innenraum 5 verlagert werden, sodass im zentrischen Bereich 43 zwischen der hinteren Membranoberfläche 42 und der Optikkörperoberfläche 21 relativ wenig Fluid vorhanden ist. Dies bewirkt eine zusätzliche Veränderung der Refraktion der Intraokularlinse 100.

### Bezugszeichen:

- 1: erster Linsenteil
- 2: Optikkörper
- 3: Haptik
- 4: Membran
- 5: Innenraum
- 10: zweiter Linsenteil
- 11: Hohlzylinder
- 12: proximales Ende des Hohlzylinders
- 13: distales Ende des Hohlzylinders
- 14: Kragen
- 15: Mittelachse des Hohlzylinders
- 21: vordere Optikkörperoberfläche
- 22: hintere Optikkörperoberfläche
- 23: Scheitelpunkt der vorderen Optikkörperoberfläche
- 24: Erhebung
- 25: optische Achse des Optikkörpers
- 41: vordere Membranoberfläche
- 42: hintere Membranoberfläche
- 43: zentrischer Bereich der Membran
- 44: peripherer Bereich der Membran
- 45: Führungselement
- 46: Mittelachse der Membran
- 47: Geschwungene Linie
- 48: Außenrand des zentrischen Bereiches der Membran
- 49: Biegeelement
- 50: Kapselsack
- 51: Innenwand des Kapselsackes
- 60: Zonulafasern
- 100: Akkommodative Intraokularlinse
- F: Druckkraft
- R: Krümmungsradius der hinteren Membranoberfläche

## Patentansprüche

1. Akkommodative Intraokularlinse (100) zur Implantation in ein Auge innerhalb eines natürlichen Kapselsackes (50) im Auge, der an seinem Umfang mittels Zonulafasern (60) an einem Ziliarmuskel des Auges angebracht ist, wobei die Intraokularlinse (100) aufweist:
- einen ersten Linsenteil (1), welcher aufweist:
- einen für Licht transparenten Optikkörper (2) mit einer optischen Achse, einer vorderen Optikkörperoberfläche (21) und einer hinteren Optikkörperoberfläche (22),
- eine mit dem Optikkörper (2) fest verbundene Haptik (3), welche eingerichtet ist, mit dem Kapselsack (50) in Eingriff zu kommen, um den Optikkörper (2) im Kapselsack (50) mittig anzuordnen,
- eine mit der Haptik (3) oder dem Optikkörper (2) fest verbundene flexible Membran (4), welche eine vordere Membranoberfläche (41) und eine hintere Membranoberfläche (42) aufweist, wobei die Membran (4) benachbart zur vorderen Optikkörperoberfläche (21) angeordnet ist, wobei die Membran (4) eine Mittelachse (46) aufweist, die kongruent oder parallel zur optischen Achse (25) verläuft, wobei die hintere Membranoberfläche (42) einen Krümmungsradius (R) aufweist, wobei die Membran (4) für Licht transparent ist, und
- einen zweiten Linsenteil (10), welcher einen Hohlzylinder (11) aufweist,
wobei sich der Hohlzylinder (11) mit einem proximalen Ende (12) auf die vordere Membranoberfläche (41) des ersten Linsenteils (1) so platzieren lässt, dass durch eine auf ein distales Ende (13) des Hohlzylinders (11) parallel zur optischen Achse (25) wirkende Druckkraft (F), welche durch eine Bewegung des Ziliarmuskels des Auges erzeugbar ist, der Hohlzylinder (11) und die Membran (4) entlang der optischen Achse (25) in Richtung zur vorderen Optikkörperoberfläche (21) hin verlagerbar sind und dabei die hintere Membranoberfläche (42) eine Änderung Ihres Krümmungsradius (R) erfährt,
**dadurch gekennzeichnet, dass** sich der Hohlzylinder (11) mit der Membran (4) lösbar koppeln lässt.

2. Intraokularlinse (100) nach Anspruch 1, wobei die Membran (4) hermetisch dicht mit der Haptik (3) oder dem Optikkörper (2) gekoppelt ist, sodass ein Innenraum (5) zwischen der hinteren Membranoberfläche (42) und der vorderen Optikkörperoberfläche (21) gebildet ist, wobei der Innenraum (5) mit Gas gefüllt ist.

3. Intraokularlinse (100) nach einem der vorherigen Ansprüche, wobei die Membran (4) Führungselemente (45) aufweist, mit welchen der Hohlzylinder (11) in Eingriff kommen kann und damit zentrisch zur optischen Achse (25) auf der Membran (4) platzierbar ist.

4. Intraokularlinse (100) nach einem der vorherigen Ansprüche, wobei die Membran (4) einen zentrischen Bereich (43) und einen peripheren Bereich (44) aufweist, wobei der zentrische Bereich (43) eine größere Dicke als der periphere Bereich (44) aufweist.

5. Intraokularlinse (100) nach einem der vorherigen Ansprüche, wobei die Membran (4) so verlagerbar ist, dass sich die hintere Membranoberfläche (42) mindestens mit einem Scheitelpunkt (23) der vorderen Optikkörperoberfläche (21) in Kontakt bringen lässt.

6. Intraokularlinse (100) nach einem der vorherigen Ansprüche, wobei der Hohlzylinder (11) an dem distalen Ende (13) einen umlaufenden Kragen (14) aufweist, der sich mit einer Innenwand (51) des Kapselsackes (50) in Eingriff bringen lässt.

7. Intraokularlinse (100) nach einem der vorherigen Ansprüche, wobei auf der vorderen Optikkörperoberfläche (21) eine Erhebung (24) ausgebildet ist, welche eine Höhe aufweist, die größer als 0,05 mm ist,

## Claims

1. Accommodative intraocular lens (100) for implantation in an eye within a natural capsular bag (50) in the eye, said natural capsular bag being attached at its periphery to a ciliary muscle of the eye by means of zonular fibres (60), the intraocular lens (100) comprising:
- a first lens part (1) comprising:
- a light-transparent optic body (2) with an optical axis, an anterior optic body surface (21) and a posterior optic body surface (22),
- a haptic (3) securely connected to the optic body (2), said haptic being configured to engage with the capsular bag (50) in order to arrange the optic body (2) in the middle of the capsular bag (50),
- a flexible membrane (4) securely connected to the haptic (3) or the optic body (2), said flexible membrane having an anterior membrane surface (41) and a posterior membrane surface (42), wherein the membrane (4) is arranged adjacent to the anterior optic body surface (21), wherein the membrane (4) has a centre axis (46) which extends congruent or parallel to the optical axis (25), wherein the posterior membrane surface (42) has a radius of curvature (R) and wherein the membrane (4) is transparent to light, and
- a second lens part (10) having a hollow cylinder (11), wherein a proximal end (12) of the hollow cylinder (11) can be placed on the anterior membrane surface (41) of the first lens part (1) in such a way that a compressive force (F), which acts on a distal end (13) of the hollow cylinder (11) parallel to the optical axis (25) and which is generable by a movement of the ciliary muscle of the eye, renders the hollow cylinder (11) and the membrane (4) displaceable along the optical axis (25) in the direction to the anterior optic body surface (21) and the posterior membrane surface (42) experiences a change in the radius of curvature (R) thereof,
**characterized in that** the hollow cylinder (11) can be detachably coupled to the membrane (4).

2. Intraocular lens (100) according to Claim 1, wherein the membrane (4) is coupled to the haptic (3) or the optic body (2) in hermetically sealed fashion such that an interior (5) between the posterior membrane surface (42) and the anterior optic body surface (21) is formed, said interior (5) being filled with a gas.

3. Intraocular lens (100) according to either of the preceding claims, wherein the membrane (4) has guide elements (45), with which the hollow cylinder (11) can engage and consequently be able to be placed on the membrane (4) centrally with respect to the optical axis (25).

4. Intraocular lens (100) according to any one of the preceding claims, wherein the membrane (4) has a central region (43) and a peripheral region (44), the central region (43) having a greater thickness than the peripheral region (44).

5. Intraocular lens (100) according to any one of the preceding claims, wherein the membrane (4) is displaceable in such a way that the posterior membrane surface (42) can be brought into contact with at least one apex (23) of the anterior optic body surface (21).

6. Intraocular lens (100) according to any one of the preceding claims, wherein the hollow cylinder (11) has a circumferential collar (14) at the distal end (13), it being possible to bring said collar into engagement with an interior wall (51) of the capsular bag (50).

7. Intraocular lens (100) according to any one of the preceding claims, wherein an elevation (24) with a height of more than 0.05 mm is formed on the anterior optic body surface (21).

## Revendications

1. Lentille intraoculaire accommodative (100) destinée à être implantée dans un œil à l'intérieur d'un sac capsulaire naturel (50) situé dans l'œil et attaché à sa périphérie à un muscle ciliaire de l'œil par des fibres zonulaires (60), la lentille intraoculaire (100) comportant :
- une première partie de lentille (1) qui comporte :
- un corps optique (2), transparent à la lumière, qui est pourvu d'un axe optique, d'une surface de corps optique avant (21) et d'une surface de corps optique arrière (22),
- une haptique (3) reliée de manière fixe au corps optique (2) et destinée à venir en engagement avec le sac capsulaire (50) afin de disposer le corps optique (2) au milieu dans le sac capsulaire (50),
- une membrane souple (4) qui est reliée de manière fixe à l'haptique (3) ou au corps optique (2) et qui comporte une surface de membrane avant (41) et une surface de membrane arrière (42), la membrane (4) étant disposée de manière adjacente à la surface de corps optique avant (21), la membrane (4) comportant un axe central (46) qui s'étend de manière congruente ou parallèle à l'axe optique (25), la surface de membrane arrière (42) ayant un rayon de courbure (R), la membrane (4) étant transparente à la lumière, et
- une deuxième partie de lentille (10) qui comporte un cylindre creux (11), le cylindre creux (11) pouvant être placé avec une extrémité proximale (12) sur la surface de membrane avant (41) de la première partie de lentille (1) de sorte qu'une force de compression (F), qui agit sur une extrémité distale (13) du cylindre creux (11) parallèlement à l'axe optique (25) et qui peut être générée par un mouvement du muscle ciliaire de l'œil, peut déplacer le cylindre creux (11) et la membrane (4) le long de l'axe optique (25) dans la direction de la surface de corps optique avant (21) et la surface de membrane arrière (42) subit une modification de son rayon de courbure (R),
**caractérisée en ce que** le cylindre creux (11) peut être accouplé de manière amovible à la membrane (4).

2. Lentille intraoculaire (100) selon la revendication 1, la membrane (4) étant accouplée de manière étanche à l'haptique (3) ou au corps optique (2) de sorte qu'un espace intérieur (5) soit formé entre la surface de membrane arrière (42) et la surface de corps optique avant (21), l'espace intérieur (5) étant rempli de gaz.

3. Lentille intraoculaire (100) selon l'une des revendications précédentes, la membrane (4) comportant des éléments de guidage (45) avec lesquels le cylindre creux (11) peut venir en engagement et donc être placé sur la membrane (4) de manière centrée par rapport à l'axe optique (25).

4. Lentille intraoculaire (100) selon l'une des revendications précédentes, la membrane (4) comportant une zone centrale (43) et une zone périphérique (44), la zone centrale (43) ayant une épaisseur supérieure à la zone périphérique (44).

5. Lentille intraoculaire (100) selon l'une des revendications précédentes, la membrane (4) pouvant être déplacée de sorte que la surface de membrane arrière (42) puisse être amenée en contact avec au moins un sommet (23) de la surface de corps optique avant (21).

6. Lentille intraoculaire (100) selon l'une des revendications précédentes, le cylindre creux (11) comportant à l'extrémité distale (13) un rebord périphérique (14) qui peut être amené en engagement avec une paroi intérieure (51) du sac capsulaire (50).

7. Lentille intraoculaire (100) selon l'une des revendications précédentes, une élévation (24) d'une hauteur supérieure à 0,05 mm étant formée sur la surface de corps optique avant (21).
